# EUROPEAN PATENT SPECIFICATION

(11) **EP 3 137 183 B1**
(45) Date of publication and mention of the grant of the patent: **23.02.2022**
(21) Application number: 15786521.3
(22) Date of filing: 13.04.2015
(51) Int. Cl.: B01D 3/36, C07C 17/38, C07C 17/383

(54) **SEPARATION OF R-1233 FROM HYDROGEN FLUORIDE**
TRENNUNG VON R-1233 AUS EINEM WASSERSTOFFFLUORID
SÉPARATION DE R-1233 CONTENU DANS DU FLUORURE D'HYDROGÈNE

(30) Priority: 29.04.2014 US 201414264374
(43) Date of publication of application: 08.03.2017
(73) Proprietor: Arkema, Inc., King of Prussia, PA 19406 (US)
(72) Inventor: WISMER, John A., Washington Crossing, Pennsylvania 18977 (US); CHEN, Benjamin Bin, Wayne, Pennsylvania 19087 (US)
(74) Representative: Arkema Patent
(86) International application number: PCT/US2015/025510
(87) International publication number: WO 2015/167784

(56) References cited:
- EP-A1- 0 940 382
- JP-A- 2014 051 485
- KR-A- 20130 041 683
- US-A- 3 947 558
- US-A1- 2008 051 612
- US-A1- 2012 010 449

## Description

The present invention relates to a separation method for isolating monochloro-trifluoropropenes such as 1,1,1-trifluoro-3-chloro-2-propene (HCFC-1233zd) from azeotropic or near azeotropic streams of monochloro-trifluoropropenes and hydrogen fluoride. The method of the present invention makes use of chilled, liquid phase separation combined with azeotropic distillations to isolate pure monochloro-trifluoropropenes such as HCFC-1233zd.

### Background of The Invention

With continued regulatory pressure there is a growing need to produce more environmentally sustainable replacements for refrigerants, heat transfer fluids, foam blowing agents, solvents, and aerosols with lower ozone depleting and global warming potentials. Chlorofluorocarbon (CFC) and hydrochlorofluorocarbons (HCFC), widely used for these applications, are ozone depleting substances and are being phased out in accordance with guidelines of the Montreal Protocol. Hydrofluorocarbons (HFC) are a leading replacement for CFCs and HCFCs in many applications; though they are deemed "friendly" to the ozone layer they still generally possess high global warming potentials. One new class of compounds that has been identified to replace ozone depleting or high global warming substances are halogenated olefins, such as hydrofluoroolefins (HFO) and hydrochlorofluoroolefins (HCFO). The HFOs and HCFOs provide the low global warming potential and zero or near zero ozone depletion properties desired.

Because of the presence of alkene linkage it is expected that the HFOs and HCFOs will be chemically unstable, relative to HCFCs or CFCs. The inherent chemical instability of these materials in the lower atmosphere results in short atmospheric lifetimes, which provide the low global warming potential and zero or near zero ozone depletion properties desired.

US Patent No. 6,013,846 discloses azeotropes of HF and 1233zd and methods for separating such azeotropes from mixtures of HF and 1233zd which are HF rich or 1233zd rich. The method comprises treating a mixture rich in HF relative to the azeotrope of 1233zd and HF in a distillation (rectification) column to obtain a distillate containing the azeotrope and a bottoms product of relatively pure HF

US 2012/010449 describes a method for producing monochloro-trifluoropropene from an azeotrope or azeotrope like combination of monochloro-trifluoropropene and HF which comprises (a) distilling a reaction mixture including hydrogen fluoride, monochloro-trifluoropropene, and hydrogen chloride to remove hydrogen chloride as overhead and a bottoms stream, (b) cooling the bottoms stream to form two liquid phases, (c) separating said two liquid phases in a liquid phase separator into a first light phase comprising hydrogen fluoride in excess over an azeotrope or azeotrope like combination of monochloro-trifluoropropene and hydrogen fluoride and a second heavy phase comprising an excess of monochloro-trifluoropropene over an azeotrope or azeotrope like combination of monochloro-trifluoropropene, (d) distilling said first light phase in a distillation column to produce a top stream of an azeotrope of monochloro-trifluoropropene and hydrogen fluoride and a bottoms stream of hydrogen fluoride, (e) and distilling said second heavy phase in a distillation train to provide a monochloro-trifluoropropene stream.

### Summary of The Invention

In the present invention, a method was discovered for separating azeotrope or near azeotrope compositions of monochloro-trifluoropropenes and HF, preferrably 1233zd and HF. The method of the present invetion is also effective in separating other isomers of 1233, such as 1233xf (1,1,1-trifluoro-2-chloro-3-propene) from azeotrope or azeotrope like combinations with HF. The azotrope or near azeotrope combination of monochloro-trifluoropropenes and HF could be produced, for example, in a liquid phase fluorination reaction of 1,1,1,3,3-pentachloropropane (240fa) or 1,1,3,3-tetrachloro-2-propene (referred to hereinafter as (1230za). The 1230za is of special interest as a starting material since it has been shown to fluorinate readily in the liquid phase without a catalyst, as taught in U.S. Pat. No. 5,877,359. One of the problems associated with the production of the trans isomer of1233zd, the preferred isomer of the present invention, is that it has nearly the same boiling point (18-20°C) as HF and azeotropes or near azeotropes can form with HF.

The fluorination of 240fa or 1230za can take place in a catalyzed or uncatalyzed liquid phase reaction. Typically the liquid phase fluorination reactor is coupled to a rectification column(s). The feed to the fluorination reactor consists of an organic chlorocarbon and HF which react to form a hydrofluorocarbon (HFC) or hydrochlorofluorocarbon (HCFC) that is more volatile than the original chlorocarbon. The HFC or HCFC product can be removed from the reaction mixture as a gas along with by-product HCl and some unreacted HF. The rectification column is coupled to the reactor to separate unreacted HF, organic and under fluorinated organic compounds from HCl. The overhead from the rectification column is an azeotrope or near azeotrope combination of 1233zd and HF which also contains the HCl by-product of the reaction. In the case of 1233zd, the organic feedstock chlorocarbon to the fluorination reactor can be 1,1,3,3 tetrachloropropene (1230za) or 1,1,1,3,3 pentachloropropane (240fa).

In the present application "distillation column" and "rectification column" are sometimes used interchangeably. Actually, however, a rectification column is a specific type of distillation column. In most distillation columns the material to be distilled is fed to the middle of the column; below the feed point is called the stripping section and above the feed point is called the rectification section. Reference is made herein to a rectification column when the material to be distilled is fed to the bottom of the "distillation column."

The process of the present invention uses azeotropic distillation columns to separate 1233zd from HF. The composition of the feed to the azeotropic distillation columns must have either HF or F 1233zd in substantial excess of its azeotropic composition. In the method of the present invention, such streams are provided by first separating, by distillation, HCl from the azeotrope or near azeotrope of 1233zd/HF that exits from the overhead of the rectification column. The azeotrope or near azeotrope combination of 1233zd/HF is than cooled to a temperature sufficient to provide separation into an HF rich phase and a 1233zd rich phase. The HF rich phase is separated from the 1233zd rich phase in a liquid phase separator. Thereafter, the HF rich phase is fed to a first azeotropic distillation column that removes the azeotrope as an overhead and pure HF as the bottoms. The 1233zd rich phase is sent to a distillation train that includes a second azeotropic distillation column. The distillation train separates the 1233zd/HF azeotrope from 1233zd via azeotropic distillation and also separates impurities from the 1233zd to provide a stream of substantially pure 1233zd.

The present invention relates to a method for producing monochloro-trifluoropropene from an azeotrope or azeotrope like combination of monochloro-trifluoropropene and HF which comprises (a) distilling a reaction mixture including hydrogen fluoride, monochloro-trifluoropropene, and hydrogen chloride to remove hydrogen chloride as overhead and a bottoms stream, (b) cooling the bottoms stream to form two liquid phases, (c) separating said two liquid phases in a liquid phase separator into a first light phase comprising hydrogen fluoride in excess over an azeotrope or azeotrope like combination of monochloro-trifluoropropene and hydrogen fluoride and a second heavy phase comprising an excess of monochloro-trifluoropropene over an azeotrope or azeotrope like combination of monochloro-trifluoropropene, (d) distilling said first light phase in a distillation column to produce a top stream of an azeotrope of monochloro-trifluoropropene and hydrogen fluoride and a bottoms stream of hydrogen fluoride, (e) contacting said second heavy phase with an alumina solid absorbent to remove HF followed by distilling said second heavy phase in a distillation train to provide a monochloro-trifluoropropene stream.

### Brief Description of the Drawings

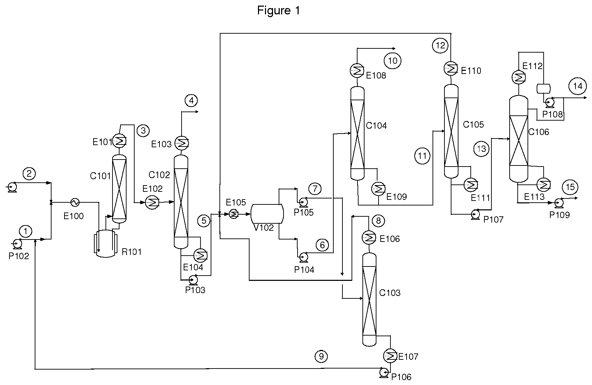

Figure 1 is a schematic of a typical process in accordance with the present invention.

### Detailed Description of The Invention

The object of the present invention is to provide a means for separating monochloro-trifluoropropenes, preferably 1,1,1-trifluoro-3-chloro-2-propene (1233zd) and more preferably the trans isomer of 1233zd referred to hereinafter as "1233zd-t", from HF when the two occur in an azeotrope or near azeotropic combinations. Azeotropic distillation is used to separate HF and 1233zd, preferably 1233zd-t, since they have very similar boiling points. The azeotropic mixture is typical of what is produced from liquid phase reactions that produce 1233zd from organic chlorocarbon feedstocks such as 1230za and 240fa. The azeotrope or near azeotrope compositions form when the reactor system uses a rectification column coupled to the reactor to separate out unreacted HF, unreacted feed organic, and under fluorinated organics from the vapor produced. The rectification column separates the vapor effluent from the reactor and produces a gas phase combination of HF and 1233zd, preferably 1233zd-t, in a ratio near the azeotropic ratio of HF and 1233zd. US Patent No. 6,013,846 discloses that this is ratio is about 2.33 moles HF per mole of 1233zd at 50°C. The overhead from such rectification columns would also contain HCl.

In accordance with the present invention, the azeotropic or near azeotropic combination of 1233zd, preferably 1233zd-t, and HF from the top of the rectification column is fed to a distillation column where HCl is removed. The HCl removal distillation column is typically operated at pressures of from about 100 psig to 300 psig. The bottoms from the HCl removal distillation column comprises the azeotrope or azeotrope like combination of 1233zd, preferably 1233zd-t, and HF. This bottoms stream is cooled sufficiently to provide that two phases form. Each phase contain the azeotrope or azeotrope like 1233zd/HF and independently HF or 1233zd. Thus the overall composition of each stream differs significantly from the azeotrope. One phase, the lighter phase, is rich in HF and the second phase, the heavier phase, is rich in 1233zd, preferably 1233zd-t,. The two phase mixture is fed to a liquid phase separator. The liquid phase separator can be operated at temperatures of from about - 60°C to +50° C, preferably from about -20° to +10 °C. The lighter liquid phase has HF in substantial excess over the azeotrope composition. This HF rich phase is sent to a first azeotropic distillation column where azeotropic 1233zd/HF is removed as overhead and relatively pure HF removed as the bottoms. The azeotropic 1233zd/HF overhead is recycled to cooled and fed to the phase separator and the HF bottoms stream is recycled to the reactor. The heavy phase from the liquid phase separator comprises a substantial excess of 1233zd, preferably 1233zd-t, over the azeotropic composition. This stream is sent to a distillation train comprising a series of distillation columns. The first distillation column removes as an overhead any very volatile impurities such as HCl or over fluorinated HFC's. The bottoms of this column is sent to a second azeotropic distillation column. This second azeotropic distillation column removes a 1233zd/HF azeotrope as overhead and crude 1233zd, preferably 1233zd-t, as a bottoms. The overhead can be recycled to be cooled and fed to the liquid phase separator. The bottoms is sent to a product recovery distillation column that recovers purified 1233zd, preferably 1233zd-t, as overhead and any heavy, organic impurities such as the cis- isomer of 1233zd as a bottoms stream. The process of the present invention provides a method whereby relatively pure 1233zd, preferably 1233zd-t, can be separated from an azeotrope or azeotrope like combination of 1233zd and HF.

Figure 1 show a schematic of a process in accordance with the present invention. The feeds to the reactor system are typically HF (Stream 1) and an organic stream, either 240fa or 1230za (Stream 2). The reactor (R101) may or may not contain a catalyst. The selective products of the reaction are 1233zd and HCl. These would exit the reaction system from the top of a rectification Column (C101) along with enough HF to be close to its azeotropic ratio with 1233zd (Stream 3).

Column C102 removes the HCl as an overhead product (Stream 4). This could be done at pressures anywhere from 100 psig to 300 psig. The bottoms from this column (stream 5) would then be cooled in a heat exchanger (E105) and sent to a liquid phase separator (V102). The liquid phase separator could operate at temperatures from -60°C to +50°C. A preferred temperature range would be -20°C to 10°C. The lighter liquid phase (Stream 7) would have HF in substantial excess over the azeotropic composition. This phase is sent to a first azeotropic distillation column (C103) that removes the HF/F1233zd azeotrope as overhead (Stream 8) and relatively pure HF as a bottoms (Stream 9). The azeotropic composition is recycled to be cooled and fed to the phase separator and the HF can be recycled to the reactor R101.

The heavy phase (Stream 6) from the phase separator contains 1233zd in substantial excess over the 1233zd/HF azeotropic composition. This stream is sent to a series of distillation columns. The first column (C104) is a purification column which removes as an overhead (Stream 10) any very volatile impurities such as residual HCl or over fluorinated HFC's. The bottoms of the first column (Stream 11) is then sent to the second azeotropic distillation column (C105). This azeotropic distillation column removes a 1233zd/HF azeotrope as overhead (Stream 12) and a crude 1233zd stream as bottoms (Stream 13). The overhead stream can be recycled to be cooled and fed to the liquid phase separator V102. The bottoms stream is sent to a product recovery distillation column (C106) that recovers pure 1233zd, preferably 1233zd-t, as overhead (Stream 14) and any organic impurities such as the cis isomer of 1233zd as a bottoms (Stream 15).

As shown in Example 1, the heavy phase (stream 6) effluent from the phase separator can have a relatively low concentration of HF. Recovery of such small amounts of HF may not be desirable. Consequently, an alternative to distillation of that stream to remove and recover the HF is to remove the HF using adsorption onto a solid adsorbent such as alumina and discard the adsorbed HF. The HF (acid) free stream remaining would flow to the first column (C104). The lighter phase (stream 7) contains a significant amount of F1233zd-t. An alternative to treatment in a dedicated azeotropic distillation column (C103) for the heaver phase is to recycle this stream to the bottom portion of column C101 (i.e. the rectification column) where the HF is separated and sent back to the reactor and the F1233zd/HF azeotrope is distilled overhead.

### Examples

### Example 1

A set of experiments were conducted to determine the liquid-liquid equilibrium in an HF-F1233zd system. A mixture of F1233zd and HF were equilibrated at four different temperatures. Samples of bottom and top phases were analyzed. The following results were obtained:

**Table 1**

| T (Deg C) | Upper Layer (wt%) | | Lower Layer (wt%) | |
|---|---|---|---|---|
| | HF | F1233zd-t | HF | F1233zd-t |
| -30 | 75.72 | 24.28 | 0.85 | 99.15 |
| -15 | 71.16 | 28.84 | 1.18 | 98.82 |
| 0 | 67.86 | 32.14 | 1.72 | 98.28 |
| 30 | 56.26 | 43.74 | 4.12 | 95.88 |

### Example 2

An example of a material balance of the relevant part of the process for a phase separator operated at -20C is shown in Table 2. The stream numbers refer to those used in Figure 1. As the table shows, the phase separation will produce two phases far enough removed from the azeotropic composition that aeotropic distillation can be used to isolate both pure HF and pure 1233zd.

| | | **Table 2** | | | | | | | |
|---|---|---|---|---|---|---|---|---|---|
| | kg/hr | | | | | | | | |
| | 5 | 6 | 7 | 8 | 9 | 10 | 11 | 12 | 13 |
| | Feed | Org Ph | HF Ph | HF Ovhd | HF Bttms | Lights | Lights | F12333zd | F1233zd |
| | | | | | | Ovhd | Btms | Ovhd | Bttms |
| | | | | | | | | | |
| 1233zd-t | 130.45 | 134.93 | 25.32 | 25.32 | 0.00 | 0.00 | 134.93 | 4.48 | 130.45 |
| 1233zd-c | 13.05 | 13.40 | 2.52 | 2.52 | 0.00 | 0.00 | 13.40 | 0.36 | 13.05 |
| 245fa | 0.00 | 0.00 | 0.00 | 0.00 | 0.00 | 0.00 | 0.00 | 0.00 | 0.00 |
| HF | 57.20 | 1.79 | 68.29 | 11.10 | 57.20 | 0.00 | 1.79 | 1.79 | 0.00 |
| HCl | 0.36 | 0.36 | 0.00 | 0.00 | 0.00 | 0.36 | 0.00 | 0.00 | 0.00 |
| Total | 201.06 | 150.48 | 96.13 | 38.93 | 57.20 | 0.36 | 150.11 | 6.62 | 143.50 |
| Temp (C) | -20 | -20 | -20 | 47 | 68 | -28 | 80 | 65 | 65 |
| Press (psia) | 40 | 40 | 40 | 65 | 66 | 130 | 130 | 50 | 88 |

## Claims

1. A method for producing monochloro-trifluoropropene from an azeotrope or azeotrope like combination of monochloro-trifluoropropene and HF which comprises (a) distilling a reaction mixture including hydrogen fluoride, monochloro-trifluoropropene, and hydrogen chloride to remove hydrogen chloride as overhead and a bottoms stream, (b) cooling the bottoms stream to form two liquid phases, (c) separating said two liquid phases in a liquid phase separator into a first light phase comprising hydrogen fluoride in excess over an azeotrope or azeotrope like combination of monochloro-trifluoropropene and hydrogen fluoride and a second heavy phase comprising an excess of monochloro-trifluoropropene over an azeotrope or azeotrope like combination of monochloro-trifluoropropene, (d) distilling said first light phase in a distillation column to produce a top stream of an azeotrope of monochloro-trifluoropropene and hydrogen fluoride and a bottoms stream of hydrogen fluoride, (e) contacting said second heavy phase with an alumina solid absorbent to remove HF followed by distilling said second heavy phase in a distillation train to provide a monochloro-trifluoropropene stream.

2. The method of claim 1 wherein said reaction mixture including hydrogen fluoride, monochloro-trifluoropropene, and hydrogen chloride is formed by reacting 1,1,1,3,3-pentachloropropane and/or 1,1,3,3-tetrachloro-2-propene and hydrogen fluoride in a reactor.

3. The method of claim 1 wherein said bottoms stream is cooled to from about -60 to +30 degrees C.

4. The method of claim 1 wherein said top stream of an azeotrope of monochloro-trifluoropropene and hydrogen fluoride is recycled to said liquid phase separator.

5. The method of claim 2 wherein said bottoms stream of hydrogen fluoride is recycled to said reactor

6. The method of claim 1 wherein said distillation train comprises a purification distillation column to separate said excess of monochloro-trifluoropropene over an azeotrope or azeotrope like combination of monochloro-trifluoropropene and hydrogen fluoride into a volatile impurities top stream and a bottoms stream which is sent to a second azeotropic distillation column to provide a top stream of an azeotrope of monochloro-trifluoropropene and hydrogen fluoride and a bottoms stream of crude monochloro-trifluoropropene and a recovery distillation column to separate said bottoms stream of crude monochloro-trifluoropropene into a top stream of purified monochloro-trifluoropropene and a bottoms stream of heavy impurities.

7. The method of claim 1 wherein said monochloro-trifluoropropene is selected from the group 1,1,1-trifluoro-3-chloro-2-propene and 1,1,1-trifluoro-2-chloro-3-propene.

8. The method of claim 6 wherein said purified monochloro-trifluoropropene is the trans isomer of 1,1,1-trifluoro-3-chloro-2-propene.

## Patentansprüche

1. Verfahren zur Herstellung von Monochlortrifluorpropen aus einem Azeotrop oder einer azeotropartigen Kombination von Monochlortrifluorpropen und HF, umfassend (a) Destillieren einer Reaktionsmischung, die für Wasserstoff, Monochlortrifluorpropen und Chlorwasserstoff enthält, zur Entfernung von Chlorwasserstoff als Kopfprodukt und eines Sumpfstroms, (b) Abkühlen des Sumpfstroms zur Bildung von zwei flüssigen Phasen, (c) Trennen der beiden flüssigen Phasen in einem Flüssigphasenscheider in eine erste leichte Phase, die Fluorwasserstoff im Überschuss gegenüber einem Azeotrop oder eine azeotropartigen Kombination von Monochlortrifluorpropen und Fluorwasserstoff umfasst, und eine zweite schwere Phase, die einen Überschuss von Monochlortrifluorpropen gegenüber einem Azeotrop oder eine azeotropartigen Kombination von Monochlortrifluorpropen und Fluorwasserstoff umfasst, (d) Destillieren der ersten leichten Phase in einer Destillationskolonne zur Herstellung eines Kopfstroms von Monochlortrifluorpropen und Fluorwasserstoff und eines Sumpfstroms von Fluorwasserstoff, (e) Inkontaktbringen der zweiten schweren Phase mit einem festen Aluminiumoxid-Absorptionsmittel zur Entfernung von HF mit anschließender Destillation der zweiten schweren Phase in einem Destillationszug zur Bereitstellung eines Monochlortrifluorpropenstroms.

2. Verfahren nach Anspruch 1, wobei die Reaktionsmischung, die Fluorwasserstoff, Monochlortrifluorpropen und Chlorwasserstoff enthält, durch Umsetzen von 1,1,1,3,3-Pentachlorpropan und/oder 1,1,3,3-Tetrachlor-2-propen und Fluorwasserstoff in einem Reaktor gebildet wird.

3. Verfahren nach Anspruch 1, wobei der Sumpfstrom auf etwa -60 bis +30 Grad C abgekühlt wird.

4. Verfahren nach Anspruch 1, wobei der Kopfstrom von einem Azeotrop von Monochlortrifluorpropen und Fluorwasserstoff zu dem Flüssigphasenscheider zurückgeführt wird.

5. Verfahren nach Anspruch 2, wobei der Sumpfstrom von Fluorwasserstoff zu dem Reaktor zurückgeführt wird.

6. Verfahren nach Anspruch 1, wobei der Destillationszug eine Reinigungsdestillationskolonne zur Trennung des Überschusses von Monochlortrifluorpropen gegenüber einem Azeotrop oder einer azeotropartigen Kombination von Monochlortrifluorpropen und Fluorwasserstoff in einen Kopfstrom von flüchtigen Verunreinigungen und einen Sumpfstrom, der einer zweiten Kolonne zur azeotropen Destillation zur Bereitstellung eines Kopfstroms von Monochlortrifluorpropen und Fluorwasserstoff und einem Sumpfstrom von rohem Monochlortrifluorpropen zugeführt wird, und eine Gewinnungsdestillationskolonne zur Trennung des Sumpfstroms von rohem Monochlortrifluorpropen in einen Kopfstrom von gereinigtem Monochlortrifluorpropen und einem Sumpfstrom von schweren Verunreinigungen umfasst.

7. Verfahren nach Anspruch 1, wobei das Monochlortrifluorpropen aus der Gruppe 1,1,1-Trifluor-3-chlor-2-propen und 1,1,1-Trifluor-2-chlor-3-propen ausgewählt wird.

8. Verfahren nach Anspruch 6, wobei es sich bei dem gereinigten Monochlortrifluorpropen um das Trans-Isomer von 1,1,1-Trifluor-3-chlor-2-propen handelt.

## Revendications

1. Procédé pour la production de monochloro-trifluoropropène à partir d'une combinaison azéotropique ou de type azéotropique de monochloro-trifluoropropène et de HF qui comprend (a) la distillation d'un mélange réactionnel comprenant du fluorure d'hydrogène, du monochloro-trifluoropropène, et du chlorure d'hydrogène pour éliminer du chlorure d'hydrogène en tant que flux de tête et un flux de produits de fond, (b) le refroidissement du flux de produits de fond pour former deux phases liquides, (c) la séparation desdites deux phases liquides dans un séparateur de phases liquides en une première phase légère comprenant du fluorure d'hydrogène en excès par rapport à une combinaison azéotropique ou de type azéotropique de monochloro-trifluoropropène et de fluorure d'hydrogène et une deuxième phase lourde comprenant un excès de monochloro-trifluoropropène par rapport à une combinaison azéotropique ou de type azéotropique de monochloro-trifluoropropène, (d) la distillation de ladite première phase légère dans une colonne de distillation pour produire un flux supérieur d'un azéotrope de monochloro-trifluoropropène et de fluorure d'hydrogène et un flux de produits de fond de fluorure d'hydrogène, (e) la mise en contact de ladite deuxième phase lourde avec un absorbant solide d'alumine pour éliminer HF suivie par la distillation de ladite deuxième phase lourde dans un train de distillation pour fournir un flux de monochloro-trifluoropropène.

2. Procédé selon la revendication 1, ledit mélange réactionnel comprenant du fluorure d'hydrogène, du monochloro-trifluoropropène, et du chlorure d'hydrogène étant formé par mise en réaction de 1,1,1,3,3-pentachloropropane et/ou de 1,1,3,3-tétrachloro-2-propène et de fluorure d'hydrogène dans un réacteur.

3. Procédé selon la revendication 1, ledit flux de produits de fond étant refroidi à une température allant d'environ -60 à +30 degrés C.

4. Procédé selon la revendication 1, ledit flux supérieur d'un azéotrope de monochloro-trifluoropropène et de fluorure d'hydrogène étant recyclé audit séparateur de phases liquides.

5. Procédé selon la revendication 2, ledit flux de produits de fond de fluorure d'hydrogène étant recyclé audit réacteur.

6. Procédé selon la revendication 1, ledit train de distillation comprenant une colonne de distillation de purification pour séparer ledit excès de monochloro-trifluoropropène par rapport à une combinaison azéotropique ou de type azéotropique de monochloro-trifluoropropène et de fluorure d'hydrogène en un flux supérieur d'impuretés volatiles et un flux de produits de fond qui est envoyé à une deuxième colonne de distillation azéotropique pour fournir un flux supérieur d'un azéotrope de monochloro-trifluoropropène et de fluorure d'hydrogène et un flux de produits de fond de monochloro-trifluoropropène brut et une colonne de distillation de récupération pour séparer ledit flux de produits de fond de monochloro-trifluoropropène brut en un flux supérieur de monochloro-trifluoropropène purifié et un flux de produits de fond d'impuretés lourdes.

7. Procédé selon la revendication 1, ledit monochloro-trifluoropropène étant choisi dans le groupe composé du 1,1,1-trifluoro-3-chloro-2-propène et du 1,1,1-trifluoro-2-chloro-3-propène.

8. Procédé selon la revendication 6, ledit monochloro-trifluoropropène purifié étant l'isomère trans du 1,1,1-trifluoro-3-chloro-2-propène.
